# EUROPÄISCHE PATENTSCHRIFT

(11) **EP 3 001 984 B1**
(45) Veröffentlichungstag und Bekanntmachung des Hinweises auf die Patenterteilung: **22.08.2018**
(21) Anmeldenummer: 15002897.5
(22) Anmeldetag: 05.11.2008
(51) Int. Cl.: A61F 2/64, A61F 2/66, A61B 5/11, A61B 5/00, A61F 2/50, A61F 2/68, A61F 2/70, A61F 2/74, A61F 2/76, A61F 5/01

(54) **VERFAHREN ZUR STEUERUNG EINES ORTHOPÄDISCHEN GELENKES**
METHOD FOR CONTROLLING AN ORTHOPAEDIC JOINT
PROCEDE DE COMMANDE D'UNE ARTICULATION ORTHOPEDIQUE

(30) Priorität: 07.11.2007 DE 102007053389
(43) Veröffentlichungstag der Anmeldung: 06.04.2016
(62) Teilanmeldung aus: 08847861.5
(73) Patentinhaber: Otto Bock HealthCare GmbH, 37115 Duderstadt (DE)
(72) Erfinder: PUSCH, Martin, 37115 Duderstadt (DE); BOITEN, Herman, 37085 Göttingen (DE); ZARLING, Sven, 37115 Duderstadt (DE)
(74) Vertreter: Stornebel, Kai

(56) Entgegenhaltungen:
- EP-A- 0 549 855
- WO-A-01/72245
- WO-A-2006/024876
- WO-A-2006/069264
- DE-A1- 19 859 931
- GB-A- 2 367 753
- US-A1- 2003 120 183
- US-A1- 2006 224 246
- US-A1- 2007 050 047

## Beschreibung

Die Erfindung betrifft ein Verfahren zur Steuerung eines orthopädischen Gelenkes einer unteren Extremität in Flexions- und/oder Extensionsrichtung mit einem verstellbaren Aktuator zur Anpassung einer orthopädischen Einrichtung an Gehsituationen, die von einem Gehen in der Ebene abweichen. Die orthopädische Einrichtung weist oberseitig Anschlussmittel an eine Gliedmaße und ein distal zu den Anschlussmitteln gelenkig angeordnetes orthopädisches Element auf.

Die DE 10 2006 021 802 als nachveröffentlichtes Dokument betrifft eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß, der an dem Prothesenkniegelenk befestigt ist, an das Treppaufgehen. Dabei wird das momentenarme Abheben des Prothesenfußes detektiert und anschließend die Flexionsdämpfung in der Anhebephase auf unterhalb eines Niveaus abgesenkt, das für ein Gehen in der Ebene geeignet ist.

Die US 2006/0224246 A1 und die US 2007/0050047 A1 beschreiben jeweils ein Verfahren zur Steuerung eines orthopädischen Gelenkes, bei dem Parameter, die Aussagen über die jeweilige Bewegung des Gelenkes machen können, gemessen und die so erhaltenen Daten zur Steuerung des Gelenkes und zum Erkennen unterschiedlicher Bewegungsabläufe verwendet werden. Gleiches gilt für die WO 2006/069264 A1.

Aus der WO 01/72245 A2, der WO 2006/024876 A2 und der EP 0 549 855 A2 sind jeweils Verfahren bekannt, mit denen unterschiedliche Phasen eines Schrittzykluses erkannt werden können. Die so ermittelten Informationen können zur Steuerung von Flexions- und Extensionsdämpfungen und der Freigabe des Knies zum freien Verschwenken verwendet werden.

Die DE 198 59 931 A1, die US 2003/0120183 A1 und die GB 2 367 753 A befassen sich ebenfalls mit Verfahren zum Steuern eines künstlichen Kniegelenkes, bei dem unter anderem auch die Dämpfung des Kniegelenkes anhand von gemessenen Parametern eingestellt werden kann.

Ausgehend von diesem Stand der Technik liegt der Erfindung die Aufgabe zugrunde, ein Verfahren zur Steuerung eines orthopädischen Gelenkes bereitzustellen, mit dem es möglich ist, besondere Gehsituationen zu berücksichtigen und ein angepasstes Verhalten der orthopädischen Einrichtung zu gewährleisten.

Erfindungsgemäß wird diese Aufgabe durch ein Verfahren mit den Merkmalen des Hauptanspruches gelöst. Vorteilhafte Ausgestaltungen und Weiterbildungen des Verfahrens sind in den Unteransprüchen aufgeführt.

Erfindungsgemäß sieht das Verfahren zur Steuerung eines orthopädischen Gelenkes, worunter sowohl Prothesen- als auch Orthesengelenke einer unteren Extremität verstanden werden, in zumindest einem Freiheitsgrad vor, dass über einen verstellbaren Aktuator eine Anpassung an verschiedene Gehsituationen vorgenommen wird, beispielsweise indem die Dämpfung in Flexions- und/oder Extensionsrichtung verändert wird. Die orthopädische Einrichtung, in der das orthopädische Gelenk integriert ist, kann dabei eine Orthese oder Prothese sein, beispielsweise ein Prothesenkniegelenk mit distalen Anschlussmitteln für einen Prothesenfuß sowie proximalen Anschlussmitteln an eine Gliedmaße. Ebenfalls ist vorgesehen, dass die orthopädische Einrichtung nur ein Prothesenfußgelenk mit einem darin befestigten Kunstfuß und Anschlussmittel an einen Unterschenkelstumpf aufweist. Neben den Exoprothesen sind auch Knie- oder Sprunggelenksorthesen vorgesehen, die mit dem Verfahren gesteuert werden können. Als distal zu den Anschlussmitteln gelenkig angeordnetes orthopädisches Element werden bei orthopädischen Kniegelenken die Unterschenkelschienen mit der Fußaufnahme bei Orthesen und der Unterschenkelschaft mit dem Prothesenfuß bei Prothesen angesehen. Bei orthopädischen Einrichtungen für das Sprunggelenk sind für Orthesen eine Fußaufnahme und Befestigungsmittel an dem Unterschenkel und bei Prothesen ein Prothesenfuß mit Anschlussmitteln an einem Unterschenkel vorzusehen.

An der orthopädischen Einrichtung sind Sensoren angeordnet, die mehrere Parameter der orthopädischen Einrichtung oder der Gliedmaße erfassen. Diese erfassten Parameter werden mit Kriterien verglichen, die anhand mehrerer Parameter und/oder Parameterverläufe erstellt worden sind. Anhand dieser Kriterien, die in einer Rechnereinheit abgelegt sind und die bestimmte Bewegungszustände oder Belastungszustände der orthopädischen Einrichtung bestimmten Gehsituationen zuordnen, wird ein bestimmtes Kriterium ausgewählt, das anhand der ermittelten Parameter und/oder Parameterverläufe für am besten passend angesehen wird. Grundsätzlich besteht die Möglichkeit, dass auch mehrere Kriterien aufgrund der ermittelten Parameter oder Parameterverläufe als geeignet angesehen werden müssen. Dann wird entweder eine Auswahl anhand zusätzlicher Kriterien getroffen oder eine Überlagerung mehrerer Sonderfunktionen ausgeübt. Die Sensoren ermitteln dabei die Parameter während des Gehens, bevorzugt während des Gehens in der Ebene, so dass der Nutzer der orthopädischen Einrichtung die Möglichkeit hat, die Sonderfunktionen einzuleiten, ohne Bewegungen ausüben zu müssen, die nicht dem natürlichen Schema entsprechen. Indem geringe Abweichungen einzelner Parameter erfasst und in Korrelation mit anderen Parametern und Abweichungen, die zu Kriterien zusammengefasst wurden, während des Gehens ausgewertet werden, ist es möglich, dass bevorstehende Bewegungsabläufe abgeschätzt werden können, so dass eine Einleitung der Sonderfunktion aus dem Gehen heraus erfolgen kann. Während es aus dem Stand der Technik bekannt ist, Sonderfunktionen durch ungewöhnlichen Bewegungsabläufe im Stand zu aktivieren, z.B. durch mehrmalige, schnelle Vorderfußbelastung oder Ausführen einer Wellenbewegung durch eine atypische, abwechselnde Belastung von Ferse und Vorderfuß, kann mit dem erfindungsgemäßen Verfahren aus dem Gehen heraus eine Umschaltung erfolgen, so dass eine "intuitive" Steuerung vorliegt, die keine bewussten Einschalthandlungen erfordert. Dies führt zu einer Erhöhung des Tragekomforts und einer Erhöhung der Sicherheit, insbesondere bei Prothesenträgern, da Fehlbedienungen minimiert oder ausgeschlossen werden.

Um Sonderfunktionen zu steuern, die von einem Gehen in der Ebene abweichen ist vorgesehen, dass Bewegungswiderstände, Bewegungsumfänge, Antriebskräfte und/oder deren Verläufe in Abhängigkeit von dem gewählten Kriterium angepasst werden. Die Steuerung der Sonderfunktion oder deren Einleitung erfolgt anhand des ausgewählten Kriteriums bzw. der ausgewählten Kriterien und umfasst z.B. ein Anpassen der Flexionsdämpfung und/oder Extensionsdämpfung auf ein Niveau, das von dem Niveau abweicht, das für ein Gehen in der Ebene geeignet ist, das Lösen einer Sperre, das Verstellen eines Antriebes und/oder Verstellen eines Anschlages. Durch eine Veränderung der Einstellung der passiven Komponenten wie Hydraulik, Bremsen oder Anschlägen wird der orthopädischen Einrichtung keine zusätzliche Bewegungsenergie zugeführt, so dass in diesem Zusammenhang auch von passiven Aktuatoren gesprochen wird. Die Verstellung der passiven Aktuatoren, z.B. Verringern eines Strömungsquerschnittes durch Verfahren eines Schiebers, erfordert auch Energie, die jedoch nicht in einer Zunahme der Bewegungsenergie der Prothese oder Orthese resultiert. Unter aktiven Aktuatoren werden Pumpen, elektrische Antriebe oder dergleichen verstanden, die aktiv den Bewegungsablauf unterstützen können. Als Aktuatoren können Schalter, Pumpen, Elektromotoren, Energiespeicher oder andere Antriebe eingesetzt werden. Als Energiespeicher sind beispielsweise Federn, Druckspeicher oder dergleichen vorgesehen, die in ihnen gespeicherte Energie gesteuert an eine Einrichtung abgeben können.

Auf diese Art und Weise ist es möglich Sonderfunktionen, z.B. ein alternierendes Treppensteigen mit einem Prothesenknigelenk auszuführen und das Treppaufsteigen für einen Prothesenträger zu erleichtern, ohne dass die Gefahr besteht, dass das versorgte Bein plötzlich wegknickt oder unter einer Treppenstufe hängen bleibt. Weiterhin ist es damit möglich, die Sonderfunktion aus verschiedenen Ausgangslagen aufrufen zu können, beispielsweise, wenn Treppaufgehen aus dem Gehen heraus, aus dem Stand, mit der versorgten Seite zuerst oder mit der kontralateralen Seite zuerst erfolgen soll, wenn die erste Stufe genommen werden soll oder die letzte Stufe erreicht ist. Ebenfalls ist es möglich, die Sonderfunktion aus einer Ausgangslage über mehrere verschiedene Kriterien aufzurufen, wenn mehrere Kriterien signifikant ein bestimmtes, zu erwartendes Gehmuster oder eine zu erwartende Gehsituation anzeigen. Dadurch wird insgesamt eine automatische Steuerung des Gelenkes ermöglicht, ohne dass bewusst von dem Nutzer der orthopädischen Einrichtung eine Handlung vorgenommen werden müsste, die von einem natürlichen Bewegungsablauf abweicht.

Damit ist es möglich, dass der Prothesen/Orthesenträger eine orthopädische Einrichtung erhält, die sich an die jeweilige Situation anpasst, ohne dass eine lange Gewöhnungsphase an die erweiterte Funktion erfolgen muss. Die Erfindung macht sich den Umstand zunutze, dass die betreffenden Ausgangslagen und Gehsituationen eine spezifische, signifikante Belastung bzw. Belastungsfolge oder Parameterfolge aufweisen, die geeignet sind, Kriterien auszubilden, um Sonderfunktionen aufzurufen und eine entsprechende Veränderung der Freiheitsgrade, insbesondere der Dämpfung wie der Flexionsdämpfung und/oder Extensionsdämpfung vorzunehmen.

Insbesondere ist eine Steuerung eines passiven Gelenkes mit der Verstellung der Dämpfung möglich, ebenfalls ist es vorgesehen, dass eine Sperre, beispielsweise eine Anschlagsperre, gelöst oder gesetzt wird oder ein Anschlag verstellt wird, so dass ein variabler Beugewinkel realisiert werden kann. Darüber hinaus ist es möglich, dass ein Antrieb verstellt wird, so dass bestimmte Elemente der orthopädischen Einrichtung aktiv verstellt werden, beispielsweise eine Unterstützung der Extensionsbewegung oder einer Flexionsbewegung im Fuß oder im Kniegelenk.

Als ein Parameter der Kriterien, die zur Auslösung einer oder mehrerer Sonderfunktionen herangezogen werden, kann die Axialkraft oder der Axialkraftverlauf, die sich in den Komponenten der orthopädischen Einrichtung einstellen, beispielsweise in der Unterschenkelschiene einer Orthese oder in dem Unterschenkelschaft einer Knieprothese, herangezogen werden. Als Kraftverlauf werden dabei ein Ansteigen oder ein Abfallen einer Axialkraft, die Änderung eines Axialkraftverlaufes und die Geschwindigkeit eines Axialkraftabfalls herangezogen. Um diese Parameter zu bestimmen, werden Kraftsensoren eingesetzt, die innerhalb der orthopädischen Einrichtung die Axialkraft bestimmen. Über ein wiederholtes Messen, bevorzugt in kurzen Zyklen, kann dann der Axialkraftverlauf bestimmt werden.

Weiterhin ist vorgesehen, dass als zu berücksichtigender Parameter bei der Auswahl von Sonderfunktionen der Gelenkwinkel oder der Verlauf der Gelenkwinkeländerung herangezogen wird. Die Gelenkwinkeländerung wird in Gestalt einer Verschwenkgeschwindigkeit oder einer Beschleunigung um die Gelenkachse während des Gehens ermittelt. Über den Gelenkwinkel kann beispielsweise die relative Position der Komponenten der orthopädischen Einrichtung bestimmt werden, wodurch es möglich wird, bestimmte Phasen des Gehens zu detektieren und daraus Rückschlüsse über die zu erwartende Gehsituation zu ziehen bzw. die zu erwartende Belastung auf die orthopädische Einrichtung zu ermitteln oder abzuschätzen.

Weiterhin können das in dem Gelenk wirkende Moment, eine Gelenkmomentenänderung oder ein Verlauf einer Gelenkmomentenänderung als Parameter heranzogen werden, da jeder Gehsituation zu einem bestimmen Zeitpunkt ein bestimmter Momentenwert zugeordnet werden kann. Über das Gelenkmoment in einer Phase selbst oder den Verlauf der Gelenkmomentänderung ist es möglich zu erkennen, ob und in welcher Gehsituation sich der Nutzer der orthopädischen Einrichtung befindet und welche zusätzlichen Dämpfereinstellungen vorgenommen werden müssen, um die nächstfolgende Gehsituation möglichst optimal zu unterstützen.

Weiterhin ist vorgesehen, dass eine Vertikalbewegung zumindest einer Komponente der orthopädischen Einrichtung erfasst wird und als Parameter zur Bestimmung eines Einleitens einer Sonderfunktion herangezogen wird, wenn dieser Parameter zusammen mit anderen Parametern innerhalb eines bestimmten Wertebereiches liegt und damit festgelegte Kriterien erfüllt. Neben einer reinen Vertikalbewegung, die ebenfalls über entsprechende Sensoren detektiert wird, kann auch ein Verlauf einer Vertikalbewegung als Parameter herangezogen werden, also die Geschwindigkeit einer Vertikalbewegung oder eine Vertikalbeschleunigung, um zu ermitteln, in welcher Ausgangslage sich der Patient befindet und welche Gehsituation zu erwarten ist.

Überraschend hat sich gezeigt, das auch eine Horizontalbewegung und/oder ein Verlauf einer Horizontalbewegung als Parameter herangezogen werden kann. Beim Treppensteigen mit einer orthopädischen Einrichtung hat sich gezeigt, dass während des Gehens ein nach hinten, also entgegen der Gehrichtung geführter Unterschenkel zuverlässige Signale liefert. Dabei steht das Knie in Laufrichtung deutlich vor dem Knöchelgelenk.

Hilfreich für die Steuerung der orthopädischen Einrichtung ist es ebenfalls, wenn ein Kippwinkel eines Teils der orthopädischen Einrichtung im Raum bestimmt wird, also, welche Neigung eine Komponente im Raum hat. Dies kann entweder über einen Absolutwinkelsensor gemessen werden, der eine Orientierung im Raum als Referenzgröße aufweist, beispielsweise die Schwerkraftrichtung, oder über eine rechnerische Ermittlung aus verschiedenen anderen Sensordaten erfolgen. Neben dem momentanen Kippwinkel ist es ebenfalls möglich, die Kippwinkeländerung und den Verlauf der Kippwinkeländerung eines Teils der orthopädischen Einrichtung als Parameter heranzuziehen, um zu entscheiden, wann und welche Sonderfunktionen bei der Dämpfung der orthopädischen Einrichtung eingeleitet werden. Der Kippwinkel kann durch die Verrechnung von Beschleunigung und Winkelgeschwindigkeit ermittelt werden, die von Beschleunigungssensoren und einem Gyroskop aufgenommen wurden. Mehrere Parameter oder Parameterverläufe werden in einem Kriterium zusammengefasst, also zwei oder mehr Parameter, um eine möglichst genaue Ermittlung des Ausgangszustandes anhand der Sensordaten und eine präzise Zuordnung und Entscheidung treffen zu können, welche Sonderfunktion eingeleitet wird. Ebenfalls ist es vorgesehen, dass mehrere Kriterien bzw. das Erfüllen mehrerer Kriterien eine Sonderfunktion einleiten können. Dadurch wird dem Umstand Rechnung getragen, dass eine Sonderfunktion, wie beispielsweise das alternierende Treppenaufgehen aus unterschiedlichen Gehgeschwindigkeiten, Positionen oder zuerst mit dem versorgten oder unversorgten Bein begonnen wird. Daher kann es vorkommen, dass genau eine Sonderfunktion eingeleitet werden muss, jedoch unterschiedliche Kriterien erfüllt sein können, damit genau diese Sonderfunktion eingeleitet wird.

Eine Weiterbildung der Erfindung sieht vor, dass das Steuerungsverfahren in der Sonderfunktion die Extensions- und/oder Flexionsdämpfung in einer Aufsetz- und Hüftstreckphase der unteren Extremität auf ein Niveau oberhalb einer Dämpfung einer Schwungphasensteuerung für das Gehen in der Ebene anhebt. Dadurch ist es möglich, dass eine kontrollierte Extension bzw. Streckung sowohl des Hüftgelenkes als auch des Kniegelenkes und des Fußgelenkes erfolgen kann.

Für das Übersteigen eines Hindernisses ist es erforderlich, dass in der Anhebephase zunächst die Flexionsdämpfung und dann die Extensionsdämpfung verringert wird, damit ein möglichst großer Schritt erfolgen kann, da ein Hindernis überwunden werden muss, beispielsweise eine Türschwelle oder ein auf dem Boden befindlicher Gegenstand, und der Fuß oder Prothesenfuß nicht auf einer nächst höheren Stufe aufgesetzt werden soll. Nach dem Aufsetzen des Fußes ist es jedoch vorgesehen, dass in der Sonderfunktion die Flexionsdämpfung und/oder Extensionsdämpfung in der Aufsetzphase auf einen maximalen Wert erhöht wird, dies gilt sowohl für das alternierende Treppensteigen als auch für das Überwinden von Hindernissen, damit das Kniegelenk beim Strecken gegen Einknicken bzw. gegen ein hartes Anschlagen in den Endanschlag gesichert ist. Ebenfalls ist es vorgesehen, dass unmittelbar vor dem Aufsetzen des Fußes oder Prothesenfußes die Extensionsdämpfung vor der Streckung erhöht wird, so dass die Positionierung des Fußes oder Prothesenfußes durch den vom Patienten direkt kontrollierbaren Hüftwinkel erfolgen kann. Eine Erhöhung der Flexionsdämpfung auf einen maximalen Wert hat den Vorteil, dass ein Einsinken bei einem nicht ausreichenden Hüftstreckmoment vermindert oder vermieden wird. Die hohe Dämpfung in der Aufsetz- und Hüftstreckphase wird dabei bevorzugt bis zur vollständigen Hüftstreckung beibehalten.

Die ursprüngliche Flexionsdämpfung innerhalb des Gelenkes kann bevorzugt in Abhängigkeit von der Veränderung des Kniewinkels angehoben werden, da über den Kniewinkel eine Vielzahl von Informationen über den Ausgangszustand ermittelt werden kann. Sobald ein festgelegter Kniewinkel erreicht wird, der in der Regel größer als ein Kniewinkel ist, der bei einer Schwungphasensteuerung für das Gehen in der Ebene geeignet ist, wird die Flexionsdämpfung angehoben. Alternativ oder ergänzend kann die Flexionsdämpfung in Abhängigkeit von der auf den Unterschenkelschaft oder auf die Unterschenkelkomponente einwirkende Axialkraft angehoben oder abgesenkt werden. Sinkt die Axialkraft in einem ausreichend schnellen Maße bis annähernd 0 bei einem nahezu gestreckten Knie, ist dies ein Indikator für das Einleiten eines Treppaufgehvorganges, so dass eine bestimmte Steuerung innerhalb der Dämpfereinrichtungen vorgenommen werden kann.

Alternativ ist es vorgesehen, dass bei einem orthopädischen Kniegelenk die Sonderfunktion bei einem auf den Unterschenkelschaft einwirkenden Axialkraftabfall und ein bestimmtes Niveau und bei einem gestreckten oder sich streckenden Kniegelenk eingeleitet wird und dabei die Flexionsdämpfung abgesenkt wird. Als zusätzlicher Parameter kann der Absolutwert der Axialkraft genutzt werden, um ein Kriterium zu definieren. Fällt die Axialkraft unter ein festgelegtes Niveau, wird die Sonderfunktion eingeleitet, wobei dann in der Sonderfunktion die Flexionsdämpfung abgesenkt wird.

Ergänzend oder alternativ ist vorgesehen, dass bei einem orthopädischen Kniegelenk die Sonderfunktion bei einem nach hinten geneigten Unterschenkel, einem gestreckten Kniegelenk und einem Kniemoment unter einem festgelegten Niveau eingeleitet wird, wobei in der Sonderfunktion die Flexionsdämpfung unter einen festgelegten Wert abgesenkt wird. Ein nach hinten geneigter Unterschenkel liegt dann vor, wenn das Kniegelenk sich deutlich vor dem Knöchelgelenk befindet.

Ebenfalls ist es vorgesehen, dass bei einer Vertikalbeschleunigung nach oben und einer Axialkraft unterhalb eines festgelegtes Niveaus die Sonderfunktion eingeleitet wird, die eine Veränderung der Flexions- und/oder Extensionsdämpfung bewirkt und dass in der Sonderfunktion die Flexionsdämpfung abgesenkt wird.

Eine Variante sieht vor, dass bei einer Horizontalbeschleunigung nach hinten, also entgegen der Gehrichtung und einer Axialkraft unterhalb eines festgelegten Niveaus die Sonderfunktion eingeleitet und in der Sonderfunktion die Flexionsdämpfung abgesenkt wird.

Die Einstellung der Flexions- und/oder Extensionsdämpfung erfolgt bevorzugt während der Anhebe- und/oder Aufsetzphase, beispielsweise wenn der Fuß oder der Prothesenfuß nach dem Anheben wieder aufgesetzt und dann eine Erhöhung der Axialkraft ermittelt wird. Ebenfalls kann bei einem annähernd konstant bleibenden Kniewinkel die Extensions- und Flexionsdämpfung angehoben werden, da dies mit einem geringen mechanischen Widerstand verbunden ist und daher relativ energiesparend durchgeführt werden kann. Die Flexionsdämpfung kann in der Anhebephase auf einem minimalen Wert abgesenkt werden, so dass die in jedem System wirksame Dämpfung aufgrund von Reibung nicht weiter erhöht wird.

Alternativ ist vorgesehen, dass die Dämpfung während der Stand- oder Schwungphase eingestellt wird.

Ebenfalls ist es möglich, dass ein momentenarmes Anheben des distal angeordneten orthopädischen Elementes über einen Kraft- oder Momentensensor ermittelt wird, wobei die Detektion mechanisch erfolgen kann, ebenso wie die Veränderung der verschiedenen Dämpfungen, um eine möglichst einfache Konstruktion der zu steuernden orthopädischen Einrichtung zu erhalten. Nach der Detektion des momentenarmen Anhebens des distalen orthopädischen Elementes kann ebenfalls die Flexionsdämpfung abgesenkt werden und zwar auf ein Niveau, das unterhalb eines Niveaus liegt, das für ein Gehen in der Ebene geeignet bzw. optimiert ist.

Durch das Absenken des Flexionswiderstandes, indem die Flexionsdämpfung abgesenkt wird, ist es möglich, einen Gelenkwinkel zu erreichen, der ein Aufsetzen eines Prothesenfußes oder eines Fußes auf einer nächsthöheren Stufe ermöglicht. Bei einer Hüftflexion und einem momentenarmen Anheben eines Prothesenfußes oder des distal angeordneten orthopädischen Elementes kann ein Kniewinkel erreicht werden, der bei einem Vorbringen der Hüfte oder bei einer entsprechenden Extension durch die Schwerkraft ausreicht, eine Treppenkante zu überwinden oder ein Hindernis zu übersteigen und den Prothesenfuß oder das distal angeordnete orthopädische Element über der Treppenstufe zu positionieren bzw. hinter dem Hindernis abzusetzen. Dabei ist es vorteilhaft, die Massenverteilung so zu gestalten, dass der Masseschwerpunkt möglichst distal angeordnet ist, so dass ohne eine Gesamtgewichtszunahme der orthopädischen Einrichtung der gewünschte Effekt der Knieflexion bei einem momentenarmen Anheben des Prothesenfußes oder des distal angeordneten orthopädischen Elementes erreicht wird.

Das Detektieren eines momentenarmen Anhebens kann über eine Messung einer Horizontalbeschleunigung des distal angeordneten orthopädischen Elementes und einer Erfassung einer Beugung innerhalb des Gelenkes erfolgen.

Grundsätzlich ist es ebenfalls vorgesehen, dass eine Flexionsunterstützung in der Anhebephase über einen vorgespannten Federmechanismus erfolgt, neben einer

Knieflexion kann auch eine Plantarflexion des Fußes erfolgen, so dass mit der Fußspitze aufgesetzt wird.

Nach erfolgter Absenkung der Flexionsdämpfung kann eine freie Extension zeitgesteuert eingestellt werden, so dass nur noch die systemimmanenten Widerstände in der Extensionsrichtung wirksam sind. Die Zeitsteuerung kann mechanisch oder elektrisch erfolgen. Die Parameter werden bevorzugt während des Gehens ermittelt, um eine Veränderung über die Aktuatoren ohne Unterbrechung des Bewegungsablaufes ermöglichen zu können.

Neben einer Steuerung in Extensions- oder Flexionsrichtung ist es ebenfalls vorgesehen, dass eine Steuerung in einem anderen Freiheitsgrad erfolgt, z.B. in Medial-Lateralrichtung, oder in Mischformen davon. Eine solche Steuerung ist beispielsweise bei Hüft- oder Knöchelgelenken sinnvoll. Auch andere rotatorische oder translatorische Freiheitsgrade können über die Steuerung beeinflusst werden.

Nachfolgend werden Ausführungsbeispiele der Erfindung anhand der Figuren näher erläutert. Es zeigen:
- Figuren 1 bis 6 -: einen schematischen Ablauf eines alternierenden Treppaufgehens mit passiver Kniegelenksprothese;
- Figur 7 -: eine schematische Darstellung des Verfahrens;
- Figuren 8 bis 10 -: Varianten des Verfahrens.

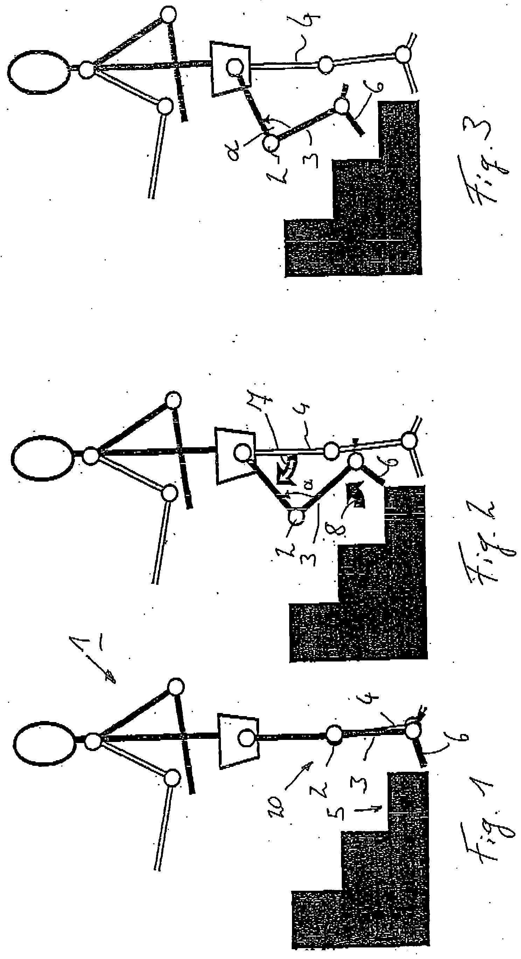

In der Figur 1 ist ein Prothesenträger 1 mit einer Kniegelenksprothese 2 dargestellt, die über oberseitige Anschlussmittel an einem Oberschenkelstumpf befestigt ist. Das Prothesenbein 20 steht mit dem gesunden kontralateralen Bein 4 vor einer Treppenstufe.

Zum Erreichen der nächsthöheren Stufe muss ein Prothesenfuß 6 um die Treppenkante geführt werden. Eine aktive Hüftbeugung, wie sie durch den Pfeil 7 angedeutet ist, unterstützt die passive Kniebeugung, die durch den Pfeil 8 gezeigt ist, die aufgrund der Massenträgheit sowohl des Prothesenfußes 6 als auch des Verbindungselementes 3 von dem Prothesenkniegelenk 2 zu dem Prothesenfuß 6 bewirkt wird. Hierzu ist eine minimale Flexionsdämpfung notwendig, damit nach einer Hüftflexion der Prothesenfuß 6 nicht nach vorne schwingt und gegen die Setzstufe oder unter die Treppenstufe 5 bewegt wird. Ziel ist es in der Abhebephase, wie sie in der Figur 2 dargestellt ist, den Prothesenfuß 6 möglichst senkrecht nach oben zu führen, gegebenenfalls eingeleitet durch eine leichte Bewegung nach hinten. Die Detektion des Anhebens erfolgt dabei über den Beugewinkel α zwischen dem Verbindungselement 3 und dem Oberschenkel oder über eine Verringerung der Axialkraft in dem Verbindungselement 3 ohne Flexion des Prothesenfußes 6. Ebenfalls ist es möglich, den Treppaufgehmodus und dadurch die Absenkung der Flexionsdämpfung auf einen Wert unterhalb der normalen Schwungphasensteuerung, bevorzugt auf den minimalen Wert, durch eine Horizontalbewegung des Prothesenfußes 6 nach hinten in Verbindung mit einer Hüftbeugung zu detektieren.

Nach dem Überwinden der Treppenkante und Beenden der Anhebephase, wie sie in der Figur 2 dargestellt ist, ist eine sichere Positionierung des Prothesenfußes 6 auf der Treppenstufe erforderlich. Dazu muss der Prothesenfuß 6 nach vorne bewegt werden, was durch Extension aufgrund der Schwerkraft erfolgen kann. Hierfür kann eine Extensionsdämpfung verringert werden, wenn dies nicht bereits in der Anhebephase erfolgt ist. Ein vor der Streckung in Flexion und Extension ausreichend bedämpftes Prothesenkniegelenk 2 ermöglicht die Positionierung des Prothesenfußes 6 durch den Prothesenträger 1, indem der Hüftwinkel verändert wird. In der Absenk- und Hüftstreckphase ist die Flexion und die Extension bevorzugt stark bedämpft, um neben der Kontrolle des Aufsetzens auch ein spontanes Zurückfallen bei einem nicht ausreichenden Hüftstreckmomentes zu verhindern. Die Extension bleibt gedämpft, um die Geschwindigkeit bei der Hüft- und Kniestreckung kontrollieren zu können. Dies ist in der Figur 3 dargestellt.

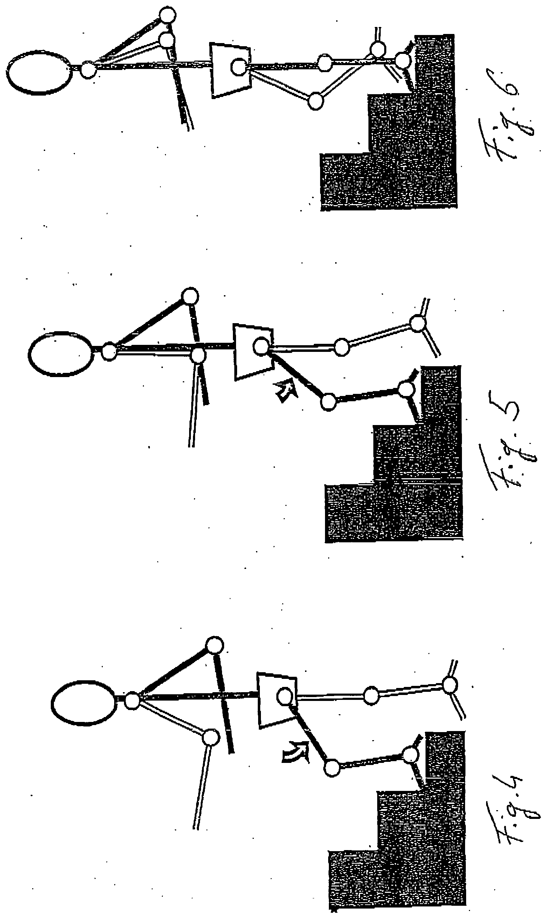

In der Figur 4 ist die Aufsetzphase abgeschlossen. Der Prothesenträger 1 kann mit einem Hüftstreckmoment die Kniestreckung einleiten. Die Kniestreckung kann durch eine Extension des gesunden Fußes unterstützt werden.

In der Figur 5 ist die zunehmende Kniestreckung durch das Aufbringen eines Hüftmomentes dargestellt. Die zunehmende Kniestreckung verkürzt den wirksamen Hebel und erleichtert die Kniestreckung durch die Hüftstreckung.

In der Figur 6 ist die vollständige Extension des mit der Kniegelenksprothese 2 versehenen Beines gezeigt. Das kontralaterale Bein 4 wird an dem Prothesenbein 20 vorbeigeführt und auf die nächsthöhere Stufe gesetzt, so dass ein alternierendes Treppaufgehen mit passiver Kniegelenksprothese möglich ist.

Die Steuerung ist demnach so eingestellt, dass während des Anhebens des Prothesenfußes 6 ein Flexionswiderstand eingestellt wird, der einen Kniewinkel α erlaubt, der das Betreten des Prothesenfußes 6 auf der nächstfolgenden Stufe ermöglicht. Eine Flexionsunterstützung durch Federmechanismen können das Anheben und das Überwinden der Stufenhöhe erleichtern.

Sollte nach der Auslösung des Treppaufgehmodus durch Detektieren eines momentenarmen Anhebens keine Aktion folgen, wird eine freie Extension eingestellt, wobei die Einstellung der freien Extension zeitabhängig erfolgt. Das Zeitglied kann auch mechanisch erfolgen. Die Detektion des momentenarmen Anhebens kann über die Massenträgheit erfolgen, wenn das gesunde Bein zunächst aufgesetzt wird und erst die zweite Treppenstufe durch das mit der Prothese versehene Bein überwunden werden soll. Erfolgt zunächst das Entlasten des Prothesenfußes und dann ein Beugen im Prothesenkniegelenk, ist das Treppaufgehen einzustellen. Die Dämpfung sowohl in Extensionsrichtung als auch in Flexionsrichtung nach der Anhebephase, also während der Hüftstreckphase, wird beibehalten, bis eine vollständige Extension des Prothesenkniegelenkes erreicht bzw. detektiert ist.

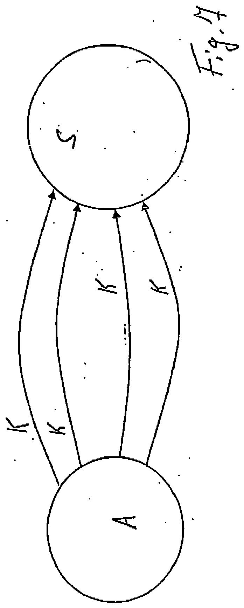

In der Figur 7 ist eine schematische Darstellung des Verfahrens gezeigt. Ausgehend von einer Ausgangslage A, die über Sensoren an der Prothese oder Orthese ermittelt wird, werden die Sensordaten mit vorgegebenen Werten oder Wertebereichen verglichen, die in einer Speichereinheit abgelegt und zu verschiedenen Kriterien K zusammengefasst wurden. Bevorzugt beschreiben mehrere Signalzustände oder Signalverläufe aus den Sensoren ein Kriterium K. Wird beispielsweise eine bestimmte Axialkraft in Verbindung mit einem Kniewinkel und einer vertikalen Bewegung gemessen, kann sich bei entsprechenden Werten der Sensoren das Kriterium ergeben, dass die Sonderfunktion S eines Überwindens eines Hindernisses einzustellen ist, was zu einer entsprechenden Verstellung der Extensions- und Flexionsdämpfung führt. Ähnlich kann bei einem schnellen Axialkraftabfall, bei einem gestreckten oder sich streckenden Knie sowie bei einer Axialkraft unter einem Niveau das Kriterium K für die Sonderfunktion S des Überwindens eines Hindernisses erfüllt werden, so dass entsprechende Dämpfereinstellungen vorgenommen werden müssen. Die ermittelten Kriterien K lösen die jeweils erforderlichen Aktionen aus, beispielsweise eine Verringerung oder Erhöhung der Flexions- und/oder Extensionsdämpfung, das Lösen einer Sperre, das Verstellen eines Antriebs oder das Justieren oder Aufheben eines Anschlages aus.

Das Überwinden von Hindernissen kann beispielsweise auch bei einem Abfallen einer Axialkraft unter ein bestimmtes Niveau und einer Kniestreckung detektiert werden, ebenfalls kann ein Kriterium durch Erfüllung einer bestimmten Neigung des Unterschenkels bzw. einer Unterschenkelschiene im Raum, einem Kniewinkel und einem geringen Kniemoment gegeben sein. Eine Vertikalbeschleunigung nach oben bei einem gestreckten Knie und einem relativ geringen Axialkraftniveau kann ebenfalls ein Kriterium für eine Sonderfunktion sein, beispielsweise das Überwinden eines Hindernisses oder das alternierende Treppensteigen.

Weiterhin ist es möglich, mehrere Signalzustände oder Verläufe, von der Ausgangslage A ausgehend, mit verschiedenen Kriterien K zu vergleichen. Der Abgleich mehrerer Kriterien K untereinander sowie mehrerer Signalzustände in einem Kriterium K stellt eine erhöhte Sicherheit zur Verfügung. Je größer die Anzahl der Signalzustände bzw. Verläufe innerhalb eines Kriteriums K ist, desto präziser ist der jeweilige Zustand der orthopädischen Einrichtung bzw. des Patienten zu ermitteln und ebenso wird dadurch die Gangsituation präziser beschrieben. Auf Grundlage dieser Information und der Signalzustände oder Verläufe innerhalb der vorgesehenen Parameter für ein Kriterium K kann dann z.B. eine gezielte Veränderung in der Dämpfercharakteristik und in dem Bewegungsablauf der orthopädischen Einrichtung vorgenommen werden.

Mehrere Kriterien K können die gleiche Sonderfunktion K auslösen, wodurch eine weitergehende Absicherung hinsichtlich einer korrekten Detektion eines Bewegungszustandes ermöglicht wird. Dies ist erforderlich, da Sonderfunktionen S wie das Übersteigen eines Hindernisses oder alternierendes Treppaufgehen oder Treppabgehen sich in ihren Bewegungsabläufen signifikant von denen des Gehens in der Ebene unterscheiden, jedoch das Erkennen dieser Sonderfunktion S aus dem Gehen in der Ebene schwierig ist. Während sich früher damit beholfen wurde, dass besonders markante Bewegungen ausgeführt wurden, um eine Sonderfunktion S einzustellen, beispielsweise ein mehrmaliges Wippen mit dem Vorderfuß, kann mit dem vorliegenden Verfahren eine automatische Anpassung des Dämpfungsverhaltens an die jeweilige Gangsituation erfolgen.

Das Gehen in der Ebene erfordert normalerweise das Verkürzen des Beines, das nach Anheben des Fußes nach vorne gesetzt werden soll. Wird das Bein nicht verkürzt, kann durch Anheben der Hüfte oder Zirkumduktion ausreichend Bodenfreiheit erzeugt werden. Physiologisch wird das Bein auch durch eine Kniebeugung verkürzt. In Beinprothesen, die das Knie und den Unterschenkel ersetzen, führen die Massenverhältnisse des Unterschenkels und die zeitlichen sowie motorischen Randbedingungen beim Gehen dazu, dass der Unterschenkel zu weit nach hinten ausschwenkt und daher die Beinprothese nicht rechtzeitig gestreckt und damit belastbar ist. Patienten gehen daher sehr langsam oder die Prothesen werden mit einer geeigneten Schwungsphasensteuerung ausgerüstet, die das Pendelverhalten des Unterschenkels deutlich dämpft. Leistungsfähige Flexionsdämpfersysteme berücksichtigen unterschiedliche Gehgeschwindigkeiten und stellen stets soviel Beinfreiheit zur Verfügung, dass die Prothesennutzer nicht stolpern, jedoch das Bein für die folgende Belastungsphase rechtzeitig wieder gestreckt ist. Je schneller der Nutzer geht, desto stärker greift die Dämpfung ein.

Zum Treppaufgehen muss ein Knie jedoch deutlich weiter als zum Gehen in der Ebene gebeugt werden, um ein Hängenbleiben an der zu überwindenden Treppenstufenkante zu verhindern. Auch zum Überwinden kleinerer Hindernisse mit einem Schritt kann die reduzierte Flexionsdämpfung genutzt werden. Eine hierfür notwendige, niedrige Flexionsdämpfung ist aus den oben genannten Gründen zum Gehen in der Ebene jedoch nicht geeignet. Zur Detektion der jeweils erforderlichen Flexionsdämpfung für den zukünftigen Schritt können mehrere Sensordaten innerhalb eines Kriteriums zusammengefasst werden und bei Erfüllung entsprechender Parameter eine Sonderfunktion auslösen. Wird die Kippung des Unterschenkels im Raum ausgewertet, kann bei einer unbelasteten Prothese oder Orthese und einem nicht gebeugten Knie ein niedriger Flexionswiderstand für das Treppaufgehen aktiviert werden. Wird das Prothesenbein bei gestrecktem Knie entlastet, kann durch die Geschwindigkeit des Lastabfalls bereits vor dem vollständigen Entlasten der Prothese ein niedriger Flexionswiderstand zum Treppaufgehen aktiviert werden. Der Nutzer hat dadurch mehr Zeit, die Beugung einzuleiten. Um zu verhindern, dass eine Flexionsdämpfung zu früh deaktiviert wird, beispielsweise wenn ein Axialkraftabfall beim Stehen in einem Fahrzeug bei holpriger Strecke detektiert wird, kann der Absolutwert der Axialkraft als zusätzlicher Parameter für ein Kriterium herangezogen werden.

Ebenfalls ist es möglich, durch die Auswertung der Oberschenkel- und Unterschenkelwinkelverläufe zueinander eine Unterscheidung zwischen Treppaufgehen und Gehen in der Ebene zu detektieren. Wird der Oberschenkel leicht nach hinten gebeugt, ist mit einer Absenkung der Flexionsdämpfung zu rechnen.

Für eine Steuerung eines Fußgelenkes ist es vorteilhaft, wenn ermittelt wird, dass sich die Zehen bzw. Vorderfußbereich nach hinten bewegt, um eine Treppenstufe oder ein Hindernis zu überwinden. Beim Aufsetzen des Hackens sollte dann keine Dorsalflexion zugelassen werden, um ein Treppaufgehen zu erleichtern. Dementsprechend wird der Widerstand nach dem Aufsetzen gegen eine Dorsalflexion erhöht. Beim Treppabgehen hingegen ist eine starke Plantarflexion beim Aufsetzen gewünscht. Um ein vollflächiges Aufsetzen des Prothesenfußes oder der Fußabnahme zu ermöglichen, sollte eine Dorsalflexion zugelassen werden, dies jedoch in einem erhöhten Maße mit einem Widerstand, um ein kontrolliertes Absetzen zu gewährleisten.

Grundsätzlich ist auch ein Streckwiderstand in der Gelenkeinrichtung, insbesondere im Kniegelenk, erforderlich, um einen Patienten zur aktiven Steuerung und zur Aktion anzuleiten. Orthopädische Kniegelenke, die ein Treppabgehen ermöglichen, sind aus dem Stand der Technik bekannt. Mittels einer hohen Dämpfung in Flexionsrichtung kann der Nutzer der orthopädischen Einrichtung auf dem Kniegelenk kontrolliert einsinken und so die nächste Treppenstufe erreichen. Die hohe Flexionsdämpfung bewirkt eine gleichmäßige Bewegung und entlastet somit die kontralaterale Seite. Beim Treppaufgehen wird in einem gesunden Kniegelenk die Bewegung durch ein kniestreckendes Moment unterstützt. Dieses Moment wird durch Muskeln bereitgestellt. Es sind orthopädische Einrichtungen bekannt, die ein Kniegelenk aufweisen, das mittels Aktuatoren eine Kniestreckung ermöglicht. Aufgrund der benötigten Energie und der auftretenden Kräfte ist ein relativ schweres und von externer Energie abhängiges Kniegelenk notwendig.

Bei Orthesen oder Prothesen der unteren Extremität ist es in vielen Fällen möglich, aus der Hüfte ein ausreichend hohes Streckmoment zu erzeugen, um das Knie beim Treppaufgehen in die Streckung zu bringen. Sobald aber das Knie anfängt zu strecken, bewegt es sich, bezogen auf den Körperschwerpunkt, nach hinten, so dass sich das kniestreckende Moment erhöht. Dieser Effekt ist selbstverstärkend. Aufgrund des sich erhöhenden, wirksamen Hebelarms erfolgt eine unkontrollierte Streckung mit einem harten, unkomfortablen Endanschlag. Um ein auf das Treppensteigen angepasstes Verhalten der orthopädischen Einrichtung ohne Zufuhr von externer Energie zu gewährleisten, ist vorgesehen, dass in der Steuerung über einen verstellbaren Aktuator nach dem Auftreten auf einer nächst höheren Treppenstufe eine signifikante Erhöhung der Streckdämpfung vorgenommen wird. Diese Dämpfung wirkt entgegen der auf das Kniegelenk wirkenden streckenden Momente und wird bevorzugt so gewählt, dass eine nahezu konstante, gut kontrollierbare Streckung ermöglicht wird. Dies kann bereits durch eine konstante Dämpfung gewährleistet werden. Ebenfalls ist es möglich, eine mit der Streckung zunehmende Dämpfung vorzusehen, um den Effekt auszugleichen, dass mit zunehmender Streckung des Kniegelenks zum Körperschwerpunkt das Kniegelenk einem größeren, wirksamen Hebelarm ausgesetzt ist.

Das Auftreten auf der nächst höheren Stufe mit einem gebeugten Kniegelenk kann wahlweise über auf der orthopädischen Einrichtung angebrachten Sensoren oder über einen mechanisch beim Auftreten betätigbaren Aktuator, wie zum Beispiel ein sich aufgrund der Axialkraft auf der orthopädischen Einrichtung im Gelenk verschiebbaren Kolben, durchgeführt werden. Die Extensionsdämpfung wird dabei auf ein Niveau angehoben, das das Niveau für ein Gehen in der Ebene deutlich übersteigt.

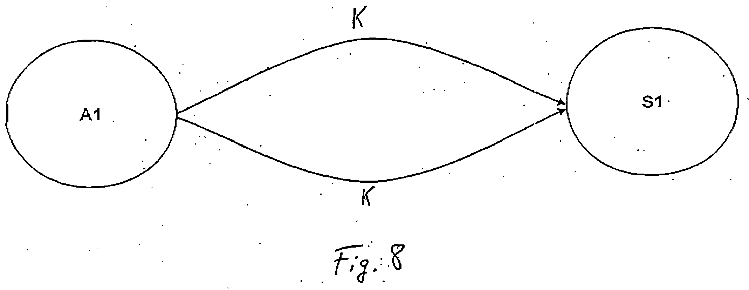

In der Figur 8 ist eine Variante der Erfindung dargestellt, bei der ausgehend von einer Ausgangslage A1 über mehrere Kriterien K eine Sonderfunktion S1 aufgerufen werden kann. Werden an der orthopädischen Einrichtung mehrere Parameter ermittelt, können unterschiedliche Parameter zu unterschiedlichen Kriterien zusammengefasst werden. Dadurch ist es möglich, dass zwei unterschiedliche Kriterien zu der Einleitung einer Sonderfunktion S führen kann.

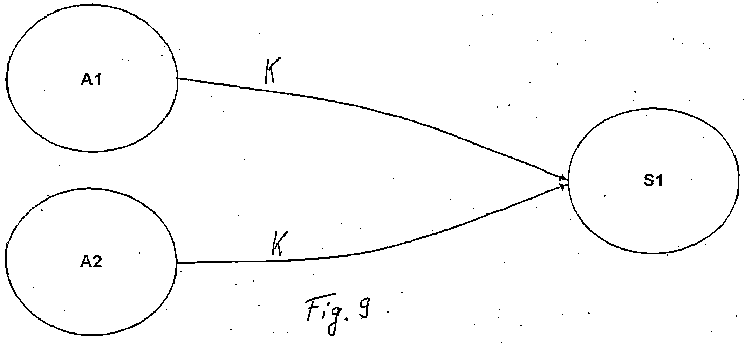

Eine Variante der Erfindung ist in der Figur 9 dargestellt in der gezeigt ist, dass aus verschiedenen Ausgangslagen A1, A2 über verschiedene Kriterien K genau eine Sonderfunktion S1 aufgerufen werden kann.

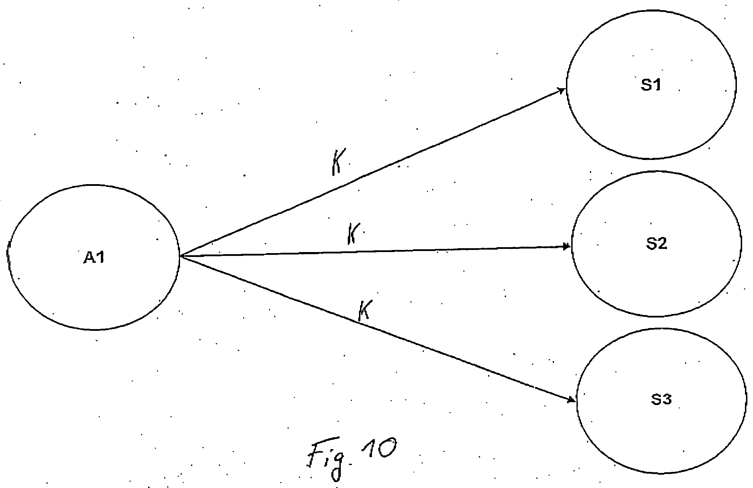

Schließlich ist in der Figur 10 dargestellt, dass aus einer Ausgangslage A1 über verschiedene Kriterien K und Parameterverläufe in verschiedene Sonderfunktionen S1, S2, S3 geschaltet werden kann. So kann es vorkommen, dass identische Momentanwerte der jeweiligen Parameter in der Ausgangssituation A1 vorliegen, beispielsweise Axialkraft, Kniewinkel und Moment, jedoch können über deren Verläufe unterschiedliche Gangsituationen prognostiziert und daher entsprechende Sonderfunktionen S1, S2, S3 eingeleitet werden, indem Antriebe zu- oder abgeschaltet, Energiespeicher freigeschaltet, Dämpfereinrichtungen variiert oder Bremsen aktiviert werden. Ebenfalls können Anschläge verstellt und Sperren gelöst werden.

## Patentansprüche

1. Verfahren zur Steuerung eines orthopädischen Gelenkes einer unteren Extremität in zumindest einem Freiheitsgrad mit einem verstellbaren Aktuator zur Anpassung einer orthopädischen Einrichtung, die oberseitige Anschlussmittel an eine Gliedmaße und ein distal zu den Anschlussmitteln gelenkig angeordnetes orthopädisches Element aufweist, an Gehsituationen, die von einem Gehen in der Ebene abweichen, mit folgenden Schritten:
a) Erfassen mehrerer Parameter der orthopädischen Einrichtung über Sensoren,
b) Vergleichen der erfassten Parameter mit Kriterien, in denen mehrere Parameter und/oder Parameterverläufe zusammengefasst wurden und die in einer Rechnereinheit abgelegt sind,
c) Auswählen eines Kriteriums, das anhand der ermittelten Parameter und/oder Parameterverläufe geeignet ist, und
d) Anpassen von Bewegungswiderständen, Bewegungsumfängen, Antriebskräften und/oder deren Verläufe in Abhängigkeit von dem gewählten Kriterium, um Sonderfunktionen zu steuern, die vom Gehen in der Ebene abweichen, **wobei** in der Sonderfunktion die Flexionsdämpfung abgesenkt wird.

2. Verfahren nach Anspruch 1, **dadurch gekennzeichnet, dass** als Aktuator ein Schalter, eine Pumpe, ein Energiespeicher und/oder ein Elektromotor eingesetzt wird.

3. Verfahren nach Anspruch 1 oder 2, **dadurch gekennzeichnet, dass** als Parameter der Gelenkwinkel und/oder der Verlauf einer Gelenkwinkeländerung und/oder die Axialkraft und/oder der Axialkraftverlauf und/oder das Gelenkmoment und/oder ein Verlauf einer Gelenkmomentänderung und/oder eine Vertikalbewegung und/oder ein Verlauf einer Vertikalbewegung und/oder eine Horizontalbewegung und/oder ein Verlauf einer Horizontalbewegung als Parameter herangezogen werden.

4. Verfahren nach einem der voranstehenden Ansprüche, **dadurch gekennzeichnet, dass** ein Kippwinkel eines Teils der orthopädischen Einrichtung im Raum und/oder ein Verlauf einer Kippwinkeländerung eines Teils der orthopädischen Einrichtung als Parameter herangezogen werden.

5. Verfahren nach einem der voranstehenden Ansprüche, **dadurch gekennzeichnet, dass** mehrere Kriterien eine Sonderfunktion einleiten können.

6. Verfahren nach einem der voranstehenden Ansprüche, **dadurch gekennzeichnet, dass** in der Sonderfunktion die Extensionsdämpfung in einer Aufsetz- und Hüftstreckphase auf ein Niveau oberhalb einer Dämpfung einer Schwungphasensteuerung für das Gehen in der Ebene angehoben wird.

7. Verfahren nach einem der voranstehenden Ansprüche, **dadurch gekennzeichnet, dass** in der Sonderfunktion die Extensionsdämpfung in der Aufsetzphase auf einen maximalen Wert erhöht wird.

8. Verfahren nach Anspruch 6 oder 7, **dadurch gekennzeichnet, dass** in der Sonderfunktion die eingestellte Extensionsdämpfung in der Aufsetz- und Hüftstreckphase bis zur vollständigen Hüftstreckung beibehalten wird.

9. Verfahren nach einem der voranstehenden Ansprüche, **dadurch gekennzeichnet, dass** bei einem orthopädischen Kniegelenk die Sonderfunktion bei einem auf den Unterschenkel einwirkenden Axialkraftabfall und einem gestreckten oder sich streckenden Kniegelenk eingeleitet wird.

10. Verfahren nach einem der Ansprüche 1 bis 8, **dadurch gekennzeichnet, dass** bei einem orthopädischen Kniegelenk die Sonderfunktion bei einem nach hinten geneigten Unterschenkel, einem gestreckten Kniegelenk und einem Kniemoment unter einem festgelegten Niveau eingeleitet wird.

11. Verfahren nach einem der Ansprüche 1 bis 8, **dadurch gekennzeichnet, dass** bei einer Vertikalbeschleunigung nach oben und einer Axialkraft unterhalb eines festgelegten Niveaus die Sonderfunktion eingeleitet wird.

12. Verfahren nach einem der Ansprüche 1 bis 8, **dadurch gekennzeichnet, dass** bei einer Horizontalbeschleunigung nach hinten und einer Axialkraft unterhalb eines festgelegten Niveaus die Sonderfunktion eingeleitet wird.

13. Verfahren nach einem der voranstehenden Ansprüche, **dadurch gekennzeichnet, dass** das Detektieren eines momentenarmen Anhebens des distal angeordneten orthopädischen Elementes über einen Kraft- oder Momentensensor oder über eine Messung einer Vertikalbeschleunigung des distal angeordneten orthopädischen Elementes und Erfassung einer Beugung im Gelenk erfolgt.

14. Verfahren nach einem der voranstehenden Ansprüche, **dadurch gekennzeichnet, dass** eine Flexionsunterstützung in der Anhebephase über einen vorgespannten Federmechanismus erfolgt.

15. Verfahren nach einem der voranstehenden Ansprüche, **dadurch gekennzeichnet, dass** nach erfolgter Absenkung der Flexionsdämpfung eine freie Extension zeitgesteuert eingestellt wird.

## Claims

1. A method for controlling an orthopedic joint of a lower extremity in at least one degree of freedom by means of an adjustable actuator with which an orthopedic appliance, which comprises upper means of attachment to a limb and an orthopedic element arranged in an articulated manner distally of the attachment means, is adapted to walking situations that deviate from walking on a plane, said method comprising the following steps:
a. detecting several parameters of the orthopedic appliance via sensors,
b. comparing the detected parameters with criteria that have been established on the basis of several parameters and/or parameter profiles and are stored in a computer unit,
c. selecting a criterion that is suitable on the basis of the detected parameters and/or parameter profiles, and
d. adapting movement resistances, movement ranges, drive forces and/or the profiles thereof in accordance with the selected criterion, in order to control special functions that deviate from walking on a plane, **wherein** in the special function the flexion damping is reduced.

2. The method as claimed in claim 1, **characterized in that** the actuator used is a switch, a pump, an energy store and/or an electric motor.

3. The method as claimed in claim 1 or 2, **characterized in that** the parameter used is the joint angle and/or the profile of a change in the joint angle and/or the axial force and/or the profile of the axial force and/or the joint torque and/or a profile of a change in the joint torque and/or a vertical movement and/or a profile of a vertical movement and/or a horizontal movement and/or a profile of a horizontal movement is used as the parameter.

4. The method as claimed in one of the preceding claims, **characterized in that** a tilt angle of a part of the orthopedic appliance in space and/or a profile of a change in the tilt angle of a part of the orthopedic appliance is used as the parameter.

5. The method as claimed in one of the preceding claims, **characterized in that** several criteria can initiate a special function.

6. The method as claimed in one of the preceding claims, **characterized in that** in the special function the extension damping in a set-down phase and a hip-straightening phase is increased to a level above a damping of a swing phase control for walking in a plane

7. The method as claimed in one of the preceding claims, **characterized in that,** in the special function, the extension damping in the set-down phase is increased to a maximum value.

8. The method as claimed in claim 6 or 7, **characterized in that,** in the special function, the adopted extension damping in the set-down phase and the hip-straightening phase is maintained until straightening of the hip is complete.

9. The method as claimed in one of the preceding claims, **characterized in that,** in the case of an orthopedic knee joint, the special function is initiated when an axial force acting on the lower leg drops and when the knee joint is straightened or being straightened.

10. The method as claimed in one of claims 1 through 8, **characterized in that,** in the case of an orthopedic knee joint, the special function is initiated when the lower leg is inclined rearward, the knee joint is straightened and a knee torque is below a fixed level.

11. The method as claimed in one of claims 1 through 8, **characterized in that** the special function is initiated when there is an upward vertical acceleration and when an axial force is below a fixed level.

12. The method as claimed in one of claims 1 through 8, **characterized in that** the special function is initiated when there is a rearward horizontal acceleration and when an axial force is below a fixed level.

13. The method as claimed in one of the preceding claims, **characterized in that** a low-torque lift of the distally arranged orthopedic element is detected via a force sensor or a torque sensor or by a measurement of a vertical acceleration of the distally arranged orthopedic element and detection of a bending in the joint.

14. The method as claimed in one of the preceding claims, **characterized in that** flexion is supported in the lift phase via a pretensioned spring mechanism.

15. The method as claimed in one of the preceding claims, **characterized in that,** after the flexion damping has been reduced, a free extension is set with time control.

## Revendications

1. Procédé pour la commande d'une articulation orthopédique d'une extrémité inférieure dans au moins un degré de liberté avec un actionneur déplaçable pour l'adaptation d'un système orthopédique, qui comprend un moyen de raccordement côté supérieur sur un membre et un élément orthopédique agencé en position distale et articulé sur le moyen de raccordement, à des situations de déambulation qui s'écartent d'une déambulation dans le plan, comprenant les étapes suivantes :
a) on détecte plusieurs paramètres du système orthopédique via des capteurs,
b) on compare les paramètres détectés avec des critères dans lesquels plusieurs paramètres et/ou plusieurs évolutions de paramètres ont été réuni(e)s, et qui sont mémorisés dans une unité de calcul,
c) on sélectionne un critère, qui convient au moyen des paramètres et/ou des évolutions de paramètres déterminé(e)s, et
d) on adapte les résistances au mouvement, les amplitudes de mouvement, les forces d'entraînement et/ou leurs évolutions en fonction du critère sélectionné, afin de commander des fonctions spéciales qui s'écartent d'une déambulation dans le plan, dans lequel, dans la fonction spéciale, l'amortissement en flexion est réduit.

2. Procédé selon la revendication 1, **caractérisé en ce que** l'on emploie à titre d'actionneur un commutateur, une pompe, un accumulateur d'énergie et/ou un moteur électrique.

3. Procédé selon la revendication 1 ou 2, **caractérisé en ce que** l'on se réfère à titre de paramètre à l'angle d'articulation et/ou l'évolution d'une variation de l'angle d'articulation et/ou à la force axiale et/ou à l'évolution de la force axiale et/ou au couple d'articulation et/ou à une évolution d'une variation du couple d'articulation et/ou à un mouvement vertical et/ou à une évolution d'un mouvement vertical et/ou à un mouvement horizontal et/ou à une évolution d'un mouvement horizontal à titre de paramètre.

4. Procédé selon l'une des revendications précédentes, **caractérisé en ce que** l'on se réfère à titre de paramètre à un angle de basculement d'une partie du système orthopédique dans l'espace et/ou à une évolution d'une variation de l'angle de basculement d'une partie du système orthopédique.

5. Procédé selon l'une des revendications précédentes, **caractérisé en ce que** plusieurs critères peuvent déclencher une fonction spéciale.

6. Procédé selon l'une des revendications précédentes, **caractérisé en ce que**, dans la fonction spéciale, on augmente l'amortissement en extension, dans une phase de dépose et dans une phase d'extension de la hanche, à un niveau au-dessus d'un amortissement d'une commande de phase d'élan pour la déambulation dans le plan.

7. Procédé selon l'une des revendications précédentes, **caractérisé en ce que**, dans la fonction spéciale, on augmente l'amortissement en extension à une valeur maximum dans la phase de dépose.

8. Procédé selon la revendication 6 ou 7, **caractérisé en ce que**, dans la fonction spéciale, on conserve l'amortissement en extension réglé dans la phase de dépose et dans la phase d'extension de la hanche jusqu'à l'extension complète de la hanche.

9. Procédé selon l'une des revendications précédentes, **caractérisé en ce que** pour une articulation orthopédique du genou la fonction spéciale est déclenchée en cas de chute de la force axiale agissant sur la cuisse et lorsque l'articulation du genou est en extension ou en cours d'extension.

10. Procédé selon l'une des revendications 1 à 8, **caractérisé en ce que** pour une articulation orthopédique du genou, la fonction spéciale est déclenchée lorsque la cuisse est inclinée vers l'arrière, que l'articulation du genou est en extension et que le couple du genou est au-dessous d'un niveau fixé.

11. Procédé selon l'une des revendications 1 à 8, **caractérisé en ce que** lors d'une accélération verticale vers le haut et pour une force axiale au-dessous d'un niveau fixé on déclenche la fonction spéciale.

12. Procédé selon l'une des revendications 1 à 8, **caractérisé en ce que** lors d'une accélération horizontale vers l'arrière et pour une force axiale au-dessous d'un niveau fixé on déclenche la fonction spéciale.

13. Procédé selon l'une des revendications précédentes, **caractérisé en ce que** la détection d'un soulèvement à faible couple de l'élément orthopédique agencé en situation distale a lieu au moyen d'un capteur de force ou de couple ou via une mesure d'une accélération verticale de l'élément orthopédique agencé en situation distale et une détection d'une flexion dans l'articulation.

14. Procédé selon l'une des revendications précédentes, **caractérisé en ce qu'**une assistance à la flexion a lieu dans la phase soulèvement via un mécanisme à ressort précontraint.

15. Procédé selon l'une des revendications précédentes, **caractérisé en ce qu'**après réduction de l'amortissement en flexion, on établit une extension libre de manière commandée dans le temps.
